# EUROPÄISCHE PATENTSCHRIFT

(11) **EP 2 424 515 B1**
(45) Veröffentlichungstag und Bekanntmachung des Hinweises auf die Patenterteilung: **22.03.2017**
(21) Anmeldenummer: 10706475.0
(22) Anmeldetag: 04.01.2010
(51) Int. Cl.: A61K 31/155, A61K 31/7028, A61P 9/00

(54) **DERIVATE VON N OMEGA-HYDROXY-L-ARGININ ZUR BEHANDLUNG VON KRANKHEITEN**
N OMEGA-HYDROXY-L-ARGININE DERIVATIVES FOR THE TREATMENT OF DISEASES
DÉRIVÉS DE N OMEGA-HYDROXY-L-ARGININE POUR LE TRAITEMENT DE MALADIES

(30) Priorität: 09.01.2009 DE 102009004203
(43) Veröffentlichungstag der Anmeldung: 07.03.2012
(73) Patentinhaber: Christian-Albrechts-Universität zu Kiel, 24118 Kiel (DE)
(72) Erfinder: CLEMENT, Bernd, 24106 Kiel (DE); SCHADE, Dennis, 24116 Kiel (DE); KOTTHAUS, Jürke, 24118 Kiel (DE)
(74) Vertreter: Steinecke, Peter
(86) Internationale Anmeldenummer: PCT/DE2010/000001
(87) Internationale Veröffentlichungsnummer: WO 2010/078865

(56) Entgegenhaltungen:
- WO-A2-03/045369
- MOALI C ET AL: "OXIDATIONS OF NG-HYDROXYARGININE ANALOGUES AND VARIOUS N-HYDROXYGUANIDINES NY NO-SYNTHASE II: KEY ROLE OF TETRAHYDROBIOPTERIN IN THE REACTION MECHANISM AND SUSTRATE SELECTIVITY" CHEMICAL RESEARCH IN TOXICOLOGY, AMERICAN CHEMICAL SOCIETY, WASHINGTON, DC, US LNKD- DOI:10.1021/TX0001068, Bd. 14, Nr. 2, 1. Februar 2001 (2001-02-01), Seiten 202-210, XP002211483 ISSN: 0893-228X
- DENNIS SCHADE ET AL: "Efficient Synthesis of Optically Pure N[omega]-Alkylated l-Arginines", SYNTHESIS, vol. 2008, no. 15, 1 August 2008 (2008-08-01), pages 2391-2397, XP055194359, ISSN: 0039-7881, DOI: 10.1055/s-2008-1067165
- KRISTIN JANSEN LABBY ET AL: 'METHYLATED NOMEGA HYDROXY-L-ARGININE ANALOGUES AS MECHANISTIC PROBES FOR THE SECOND STEP OF THE NITRIC OXIDE SYNTHASE-CATALYZED REACTION' BIOCHEMISTRY Bd. 52, Nr. 18, 07 Mai 2013, Seiten 3062 - 3073, XP055194376 DOI: 10.1021/bi301571v ISSN: 0006-2960
- DENNIS SCHADE ET AL: 'Prodrug design for the potent cardiovascular agent N[omega]-hydroxy-l-arginine (NOHA): Synthetic approaches and physicochemical characterization' ORGANIC & BIOMOLECULAR CHEMISTRY Bd. 9, Nr. 14, 5249, 01 Januar 2011, Seiten 5249 - 5259, XP055194373 DOI: 10.1039/c0ob01117g ISSN: 1477-0520

## Beschreibung

Die vorliegende Erfindung betrifft physikalisch-chemisch und pharmakokinetisch verbesserte Derivate von *N*^{ω}-Hydroxy-L-arginin (NOHA), sowie ein Verfahren zur Herstellung der erfindungsgemäß in ihren physikalisch-chemischen und pharmakokinetischen Eigenschaften verbesserten NOHA-Derivate.

*N*^{ω}-Hydroxy-L-arginin stellt das physiologisch vorkommende Intermediat der NO-Synthasekatalysierten Oxidation von L-Arginin dar. NOHA wird in einem weiteren Schritt durch die NO-Synthase oxidiert, wobei Stickstoffmonoxid freigesetzt und L-Citrullin gebildet wird. Somit ist die semi-essenzielle Aminosäure L-Arginin die natürliche Quelle für das Stickstoffmonoxid (NO).

Stickstoffmonoxid (NO) ist u.a. für die Blutversorgung der Organe von entscheidender Bedeutung. NO führt indirekt zu einer Vergrößerung der Gefäße. Das Ausmaß der Gefäßerweiterung hat dann in den einzelnen Organen unterschiedliche Wirkungen haben. Im Herzen kommt es beispielsweise zu einer besseren Durchblutung. Neben der Weitstellung der Gefäße hat NO auch noch andere Eigenschaften:
- Die erschlaffende Wirkung betrifft nicht nur die Muskulatur der Gefäße, sondern auch die des Bronchialbaums.
- NO, das von den Endothelzellen in das Gefäßlumen abgegeben wird, kann die Zusammenlagerung von Blutplättchen verhindern (= Thrombusbildung).
- Im Nervensystem dient es als wichtiger Signalstoff (Transmitter), der Einfluss auf Gehirn- und Magen-Darm-Funktionen nimmt. So rufen in der Darmwand gelegene Nervenendigungen durch die NO-Ausschüttung eine Relaxierung (Entspannung) der Ringmuskulatur hervor.
- In Abwehrzellen (Makrophagen) wird NO gebildet, das in der Lage ist, Bakterien zu zerstören. Angeregt von Bakterienbestandteilen (z.B. Lipopolysacchariden) produzieren Makrophagen NO in hohen Konzentrationen, so dass lebenswichtige z.B. eisenhaltige Enzyme blockiert werden. Die Konservierung von Fleisch durch Pökeln beruht genau auf diesem Vorgang.

Eine Dysregulation oder verminderte NO-Verfügbarkeit steht somit in Zusammenhang mit diversen kardiovaskulären Erkrankungen. Eine eingeschränkte NO-Verfügbarkeit wird daher mit der sog. endothelialen Dysfunktion assoziiert - ein Zustand multifaktorieller Genese - die mit Bluthochdruck, Atherosklerose, arterieller Thrombose, koronarer Herzkrankheit, Herzinsuffizienz, Herzinfarkt, Hypercholesterolämie und Diabetes in Verbindung gebracht wird.

Alte, starre, atherosklerotisch veränderte Gefäße können sich mit NO wieder verformen. Die Durchblutung wird dadurch verbessert, und u.a. Bluthochdruck kann sich normalisieren. Bei Kindern, die mit schweren Atemstörungen auf die Welt kommen, wird heute schon erfolgreich die Einatmung (Inhalation) von NO angewandt. NO fördert die Erektion des Penis, was zu der Entwicklung von Medikamenten gegen die Impotenz geführt hat (Viagra®).

Auch bei der Tumorbekämpfung verspricht man sich, neue Wege mit NO gehen zu können, da NO, das von weißen Blutzellen produziert wird, nicht nur Bakterien, sondern auch Zellen unschädlich machen kann.

Die Lebensdauer von NO ist allerdings nicht sehr lange. Innerhalb kurzer Zeit reagiert es mit Sauerstoff-Molekülen zu Nitrit (NO₂⁻) und Nitrat (NO₃⁻). Die Kurzlebigkeit erklärt auch, warum NO immer nur unmittelbar an seinem Wirkort gebildet werden kann.

Des Weiteren ist bekannt, dass NOHA einen potenten Inhibitor der Arginase I darstellt mit einem *K*ᵢ-Wert zwischen 30-42 µM.

In kleineren *in* vivo-Studien mit Ratten konnten mit einer i.v.-NOHA-Therapie gewünschte Effekte zur Behandlung der erektilen Dysfunktion, endothelialen Dysfunktion und Hypertension bereits demonstriert werden.

Die Behandlung von Erkrankungen, die mit endothelialer Dysfunktion assoziiert sind, mit den konventionellen NO-Donoren bringt einige Nachteile mit sich. Bei den Nitraten wären in diesem Zusammenhang z.B. die kurze therapeutische Halbwertszeit, die geringe orale Bioverfügbarkeit, teilweise adverse hämodynamische Effekte und Toleranzerscheinungen zu nennen. Tatsächlich wird eine proatherogene Wirkung bei langfristiger Behandlung mit organischen Nitraten in jüngster Zeit diskutiert.

Um eine nebenwirkungsarme Therapie von NO-defizienten Erkrankungen zu ermöglichen, muss die physiologische Situation möglichst gut imitiert werden, d.h. NO nur für eine kurze Zeit, in den richtigen Mengen und am richtigen Ort freigesetzt werden. All diese Voraussetzungen sind mit NOHA als NO-Donor erfüllt, bzw. um den Faktor Zeit zu berücksichtigen, mit einem retardierenden Prodrug von NOHA. Neben der Tatsache, dass über diese Strategie Stickstoffmonoxid ausschließlich dort freigesetzt wird, wo es benötigt und in zu geringem Ausmaß gebildet wird, kann ausgenutzt werden, dass NOHA einen der potentesten Arginase-Hemmstoffe darstellt. Gerade eine erhöhte Arginase-Aktivität wird als Mechanismus der verminderten NO-Verfügbarkeit und damit als beteiligter Faktor bei der Entstehung der endothelialen Dysfunktion diskutiert. Es wird also ein dualer Wirkungsmechanismus mit NOHA ausgenutzt.

Die Verwendung von L-Arginin oder *N*^{ω}-Hydroxy-L-arginin sowie deren einfache Carbonsäureester, als auch analoge *N*-Hydroxyguanidine zur Behandlung einer Vielzahl von Erkrankungen sind beschrieben und patentrechtlich geschützt (eine Auswahl: WO03045369, US6277884, WO0132167, CA02386938).

In der Praxis wird der Einsatz derartiger Verbindungen aber durch ihr schlechtes pharmakokinetisches Profil limitiert. Die unzureichenden Arzneistoffqualitäten dieser Substanz sind vor allem durch das Vorhandensein einer unsubstituierten *N*-Hydroxyguanidin-Funktion zu erklären. Im einzelnen sind folgende Effekte bei der physikalisch-chemischen Instabilität von *N*-Hydroxyguanidinen zu betrachten:
*N*-Hydroxyguanidine zersetzen sich bei Raumtemperatur und sollten bei 4-8 °C, besser -20 °C gelagert werden. Am stabilsten sind sie in Form ihrer Salze starker Säuren. Die folgenden Zersetzungsprozesse sind bekannt:
   (1) Hydrolyseempfindlichkeit: Besonders bei höheren pH-Werten (> pH 7) erfolgt Umsetzung zu Cyanamiden und Hydrolyse zu Harnstoffen, was unter Berücksichtigung des physiologischen pH-Wertes von 7,4 *in vivo* relevant ist.
   (2) Oxidationsempfindlichkeit: Die Oxidationsanfälligkeit stellt das wohl größte Problem dieser Stoffklasse dar, da mit vielen sehr unterschiedlichen Oxidationsmitteln gezeigt werden konnte, dass Zwei- und Dreielektronen-Oxidationen möglich sind. Mit einfach-substituierten aliphatischen und aromatischen Hydroxyguanidinen wurden Oxidationspotentiale von Eₒₓ₁ = +0,51-0,62 V und Eₒₓ₂ = +1,14-1,81 V ermittelt. Die Zersetzungsprodukte können in Abhängigkeit vom Oxidationsmittel unterschiedlich sein. Auch Metallkationen wie beispielsweise Eisen(II)(III) oder Kupfer(II) begünstigen derartige Prozesse, bzw. sind daran beteiligt. Diese Vorgänge sind von physiologischer Relevanz, da bekannt ist, dass *in vivo* oxidative Prozesse dominieren und eine ganze Reihe physiologischer Substanzen (reaktive Sauerstoffspezies, Metallkationen) diese Oxidationen begünstigen.

Metabolische Instabilität von *N*-Hydroxyguanidinen:
*N*-Hydroxyguanidine werden im Körper enzymatisch-katalysiert effektiv zu den korrespondierenden Guanidinen reduziert. Die dafür verantwortlichen Enzymsysteme wurden schon teilweise identifiziert. So ist die mitochondriale *N*-Reduktion von Cytochrom b5, Cytochrom b5-Reduktase und einem Molybdän-Cofaktor-abhängigen Enzym (mARC) abhängig. Die mikrosomale *N*-Reduktion wird durch Cytochrom b5, Cytochrom b5-Reduktase und einer dritten bislang unbekannten Komponente katalysiert. Zusätzlich ist bekannt, dass Hydroxyguanidine im Rahmen einer metabolischen Phase-II-Biotransformation *O*-glucuronidiert werden können, um in eine besser ausscheidbare Form überführt zu werden.

Dieses führt zu einer starken Limitierung der biologischen Halbwertszeit durch thermische, hydrolytische, oxidative und enzymatische Prozesse. Auch ein hoher *first-pass*-Effekt ist zu erwarten, unter Berücksichtigung der angesprochenen metabolischen Instabilität.

Daher ist es Aufgabe der Erfindung die physikalisch-chemische und metabolische Stabilität und damit die pharmakokinetischen Eigenschaften von *N*^{ω}-Hydroxy-L-arginin sowie deren einfache Carbonsäureester, als auch analoge *N*-Hydroxyguanidine zu verbessern.

Die Aufgabe wird gelöst durch die Verwendung mit den Merkmalen von Anspruch 1. Die Unteransprüche geben vorteilhafte Ausgestaltungen der Erfindung wieder.

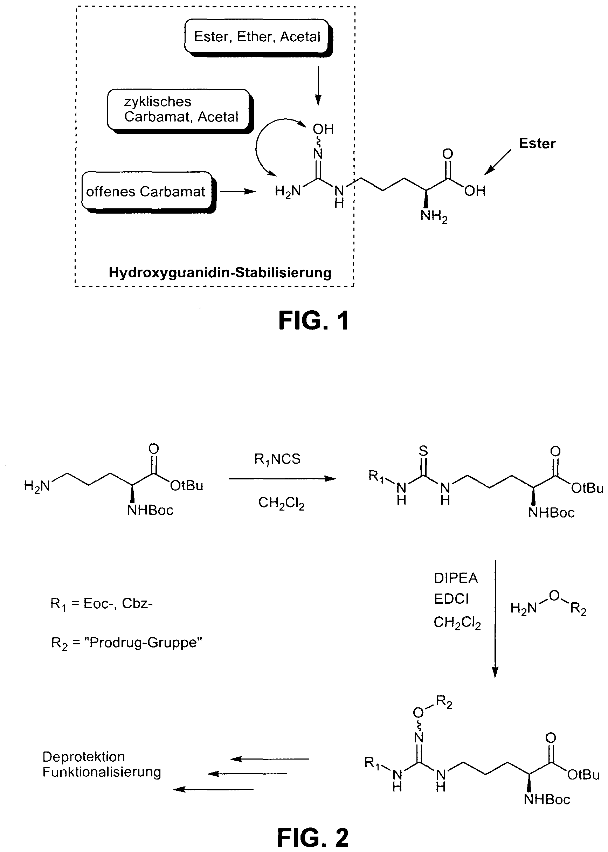

Durch die erfindungsgemäße Substitution der Hydroxyguanidin-Funktion wurden überraschenderweise erstmals thermo- und oxidationsstabile Derivate des NOHA erhalten. Die Abbildung 1 zeigt eine schematische Übersicht über die erfindungsgemäße Substitution der NOHA-Derivate.

Im Folgenden ist die chemische Formel der erfindungsgemäßen Derivate gezeigt.

Die erfindungsgemäßen Substitutionen sind mit einem X gekennzeichnet. Sowohl eine Substitution am Sauerstoff der Hydroxylgruppe X₂ führt zu überraschend stabilen Derivaten, wie auch eine Substitution am Stickstoff X₁ sowie auch eine Kombination der beiden erfindungsgemäßen Substitutionen. Die Carbonsäurefunktion kann dabei in üblicher Weise mit einem Alkyl- oder Arylrest verestert sein oder als Säure vorliegen (R₁). Y kann einem Wasserstoff, Alkoxycarbonyl- oder Aryloxycarbonylrest entsprechen. n kann 2 oder 3 betragen. Das Chiralitätszentrum m kann *R*- oder *S*-konfiguriert sein. 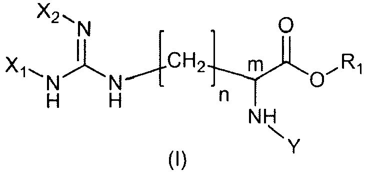 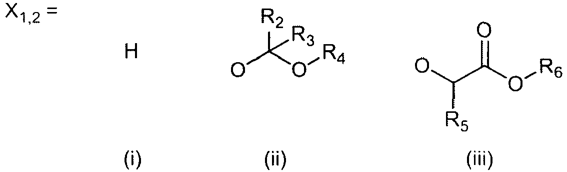 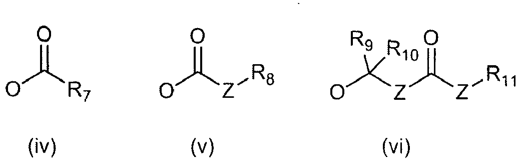 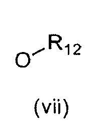 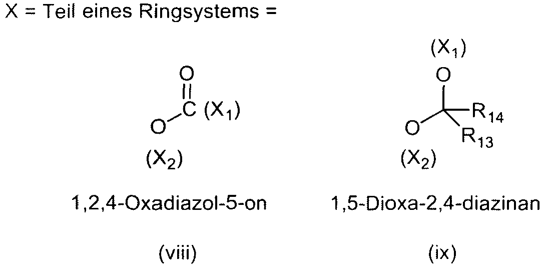 wobei
R₁ einem Wasserstoff, Alkyl- oder Arylrest entsprechen kann; X₁ und X₂ identisch oder unterschiedlich sein können und den Strukturen (i-vii) entsprechen (Ausnahme ist (i) bei dem X₁ und X₂ unterschiedlich sein müssen; auch ausgenommen ist *N*^{ω}-Hydroxy-L-arginin selbst und seine Carbonsäureester), wobei R₂, R₃ und R₄ Wasserstoff, Alkyl- oder Arylresten entsprechen können oder so gestaltet sind, dass sie in ihrer Struktur einem Monosaccharid(derivat) entsprechen und R₅₋₁₂ einem Wasserstoff, Alkyl- oder Arylrest entsprechen können; n einer Anzahl an Methylen (CH₂)-Gruppen von 2-3 entspricht; das Chiralitätszentrum m *R*- oder *S*-konfiguriert sein kann; Y einem Wasserstoff, Alkoxycarbonyl- oder Aryloxycarbonylrest entsprechen kann; Z einem Sauerstoff- oder einem Stickstoff-Atom entsprechen; X_{1,2} so gestaltet sind, dass sie Teil von Ringsystemen der Struktur (viii) oder (ix) werden, wobei R₁₃ und R₁₄ Wasserstoff, Alkyl- oder Arylresten entsprechen können

In Abbildung 1 ist eine übersichtliche Darstellung der erfindungsgemäßen Optimierung der Arzneistoffeigenschaften von *N*^{ω}-Hydroxy-L-arginin (NOHA) als NO-Donor und Arginase-Hemmstoff zur Behandlung von Krankheiten gezeigt.

Für die Synthese der erfindungsgemäßen Derivate des *N*^{ω}-Hydroxy-L-arginins wurde ein Konzept entwickelt, welches die Darstellung sehr unterschiedlich substituierter Verbindungen ermöglicht (siehe Abbildung 2). Exemplarisch, die Allgemeinheit der Lehre nicht einschränkend, ist die Synthese unterschiedlich gearteter NOHA-Derivate in den Ausführungsbeispielen beschrieben.

### Material und Methoden

### Ausführungsbeispiel 1: O-Alkylierte NOHA-Derivate

### N^{ω}-Benzyloxycarbonyl-N^{α}-(t-butyloxycarbonyl)-L-thiocitrullin-t-butylester (1)

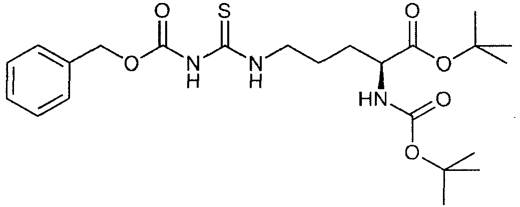

Allgemeine Vorschrift in Anlehnung an Linton et al. [J. Org. Chem. 2000, 65, 1566]: 7,0 mmol *N*^{α}-(*t*-Butyloxycarbonyl)-L-ornithin-*t*-butylester werden in 250 mL trockenem Dichlormethan gelöst. Die Lösung wird auf 0 °C gekühlt und 14 mL einer 0,5 M Lösung (in Dichlormethan) Benzyloxycarbonylisothiocyanat (7,0 mmol) tropfenweise über einen Zeitraum von 30 min zugegeben. Das Reaktionsgemisch wird zwei Stunden gerührt, wobei die Lösung auf Raumtemperatur aufwärmt. Anschließend wird das Gemisch am Rotationsverdampfer auf ca. ein Drittel des ursprünglichen Volumens i. Vak. eingeengt und mit jeweils 25 mL 1 %-iger HCl, Wasser und gesättigter NaCl-Lösung gewaschen. Die organische Phase wird über Na₂SO₄ getrocknet und am Rotationsverdampfer entfernt. Der Thioharnstoff 1 ist an dieser Stelle meist schon zu > 96 % rein (DC) und wird mittels Säulenchromatographie weiter aufgereinigt.

Nach Säulenchromatographie über Kieselgel (Cyclohexan/Ethylacetat, 4:1) wird ein hellgelbes Öl erhalten, welches im Kühlschrank fest wird.

| | |
|---|---|
| Ausbeute: | 3,13 g (93 %) |
| DC: | R_{f} = 0,31 (Cyclohexan/Ethylacetat, 4:1; Ninhydrin) |

¹H-NMR (CDCl₃):
δ/ppm = 1.44, 1.46 (s, 9H, 2 × C(CH₃)₃), 1.64-1.75 (m, 4H, β,γ-CH₂), 3.66 (m, 2H, N-CH₂), 4.20 (m, 1H, α-CH), 5.08 (m, 1H, NH), 5.10 (s, 2H, CH₂-Cbz), 7.36 (m, 5H, ArH), 8.15 (br s, 1H, NH), 9.65 (br s, 1H, NH).
¹³C-NMR (CDCl₃):
δ/ppm = 24.8 (γ-CH₂), 28.7, 29.0 (2 × C(CH₃)₃), 30.9 (β-CH₂), 45.8 (N-CH₂), 54.2 (α-CH), 68.8 (CH₂-Cbz), 80.4, 82.8 (2 × C(CH₃)₃), 129.0, 129.4, 129.5 (ArCH), 135.2 (ArC), 153.2 (CO-Cbz), 156.0 (CO-Boc), 172.2 (COO*^{t}*Bu), 179.8 (C=S).
MS (ESI):
m/z = 482 [M + H]⁺, 426 [M - C₄H₈ + H]⁺, 370 [M - 2 × C₄H₈ + H]⁺, 326 [M - 2 × C₄H₈ - CO₂ + H]⁺.
C₂₃H₃₅N₃O₆S (481.61)
Ber. C 57.36 H 7.33 N 8.73
Gef. C 57.55 H 7.60 N 8.68

### N^{α}-(t-Butyloxycarbonyl)-N^{ω}-ethoxycarbonyl-L-thiocitrullin-t-butylester (2)

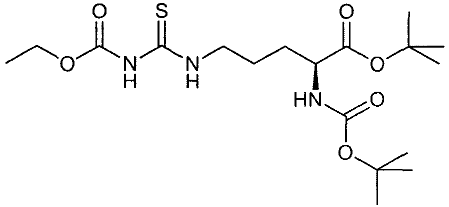

8,0 mmol *N*^{α}-(*t*-Butyloxycarbonyl)-L-omithin-*t*-butylester werden in 250 mL trockenem Dichlormethan gelöst. Die Lösung wird auf 0 °C gekühlt und 927 mg Ethoxycarbonylisothiocyanat (7,0 mmol), gelöst in 15 mL trockenem Dichlormethan, tropfenweise über einen Zeitraum von 30 min zugegeben. Das Reaktionsgemisch wird zwei Stunden bei Raumtemperatur gerührt. Anschließend wird das Gemisch am Rotationsverdampfer auf ca. ein Drittel des ursprünglichen Volumens eingeengt und mit jeweils 25 mL 1 %-iger HCl, Wasser und gesättigter NaCl-Lösung gewaschen. Die organische Phase wird über Na₂SO₄ getrocknet und i. Vak. eingeengt. Der Thioharnstoff **2** ist an dieser Stelle meist schon zu > 96 % rein (DC). Das Produkt wird im Fließmittel angelöst, mit Aktivkohle versetzt und mittels Flashchromatographie über eine kurze Kieselgel-Säule gereinigt. Es wird eine Säule mit etwa 20 g Kieselgel und als Elutionsmittel Cyclohexan/Ethylacetat (4:1) verwendet.

| | |
|---|---|
| Ausbeute: | 2,76 g eines farblosen Öls (94 %) |
| DC: | R_{f} = 0,31 (Cyclohexan/Ethylacetat, 4: 1;Ninhydrin) |
| Schmp.: | 81 °C |

¹H-NMR (CDCl₃):
δ/ppm = 1.31 (t, 3H, ³*J* = 7.1 Hz, CH₂-CH₃), 1.45, 1.47 (2 × s, 9H, C(CH₃)₃), 1.61-1.90 (m, 4H, β,γ-CH₂), 3.66 (pseudo q, 2H, N-CH₂), 4.22 (q, 2H, ³*J* = 7.1 Hz, CH₂-CH₃), 5.08 (m, 1H, α-CH), 8.06, 9.70 (2 × br s, 1H, NH).
¹³C-NMR (CDCl₃): δ/ppm = 14.9 (CH₂-CH₃), 24.9 (γ-CH₂), 28.7, 29.0 (2 × C(CH₃)₃), 31.0 (β-CH₂), 45.8 (N-CH₂), 54.3 (α-CH), 63.4 (O-CH₂), 82.8 (C(CH₃)₃), 153.4 (CO-Eoc), 156.2 (CO-Boc), 172.5 (COO'Bu), 180.1 (C=S).
MS (ESI):
m/z = 442 [M + Na]⁺, 420 [M + H]⁺, 308 [M - 2 × C₄H₈ + H]⁺, 264 [M - 2 × C₄H₈ - CO₂ + H]⁺.
C₁₈H₃₃N₃O₆S (419.54)
Ber. C 51.53 H 7.93 N 10.02 S 7.64
Gef. C 51.68 H 7.97 N 10.06 S 7.62

### N^{ω}-Benzyloxycarbonyl-N^{α}-(t-butyloxycarbonyl)-N^{ω'}-methoxy-L-arginin-t-butylester (3)

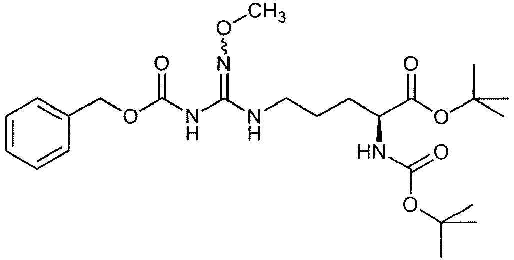

Es werden 0,5 mmol des Thioharnstoffs 1 in 5 mL trockenem Dichlormethan gelöst und 522 µL DIPEA (3 mmol) sowie 1,5 mmol Hydroxylammonium Hydrochlorid zugegeben. Die Lösung wird für ca. 30 min auf 0 °C gebracht und 287 mg *N*-Ethyl-*N'*-(3-dimethylaminopropyl)carbodiimid Hydrochlorid (1,5 mmol) zugegeben. Sofern nicht anders angemerkt, wird das Gemisch über Nacht bei Raumtemperatur gerührt. Die Lösung wird mit ca. 10 mL Dichlormethan verdünnt und jeweils mit 5 mL 1 %-iger HCl, Wasser und gesättigter NaCl-Lösung gewaschen. Die organische Phase wird über Na₂SO₄ getrocknet und am Rotationsverdampfer eingeengt. Es resultieren meist Öle, die mittels Flashchromatographie über Kieselgel weiter aufgereinigt werden. Die verwendeten Elutionsmittel und erzielten Ausbeuten sind bei den jeweiligen Substanzen angegeben.

Als Elutionsmittel wird Dichlormethan/Methanol (99:1) verwendet.

| | |
|---|---|
| Ausbeute: | 235 mg eines farblosen Öls (95 %) |
| DC: | R_{f} = 0,30 (Dichlormethan/Methanol, 99:1;Ninhydrin) |

¹H-NMR (CDCl₃):
δ/ppm = 1.44, 1.46 (2 × s, 9H, C(CH₃)₃), 1.58-2.02 (m, 4H, β,γ-CH₂), 3.11 (m, 2H, N-CH₂), 3.66 (s, 3H, O-CH₃), 4.18 (m, 1H, α-CH), 5.11 (m, 1H, NH), 5.13 (s, 2H, CH₂-Cbz), 6.25 (m, 1H, NH), 7.36 (m, 5H, ArH), 7.91 (br s, 1H, NH).
¹³C-NMR (CDCl₃):
δ/ppm = 25.6 (γ-CH₂), 28.7, 29.0 (2 × C(CH₃)₃), 30.9 (β-CH₂), 41.2 (N-CH₂), 54.5 (α-CH), 62.0 (O-CH₃), 68.3 (CH₂-Cbz), 80.3, 82.5 (2 × C(CH₃)₃), 129.0, 129.3, 129.4 (ArCH), 135.8 (ArC), 148.8 (C=N), 153.6 (CO-Cbz), 156.0 (CO-Boc), 172.5 (COO*^{t}*Bu).
MS (ESI): m/z = 517 [M + Na]⁺, 495 [M + H]⁺, 439 [M - C₄H₈ + H]⁺.
C₂₄H₃₈N₄O₇ (494.58)
Ber. C 58.28 H 7.74 N 11.33
Gef. C 58.72 H 7.99 N 11.22

### N^{ω}-Methoxy-L-arginin Bis(trifluoracetat) (3)

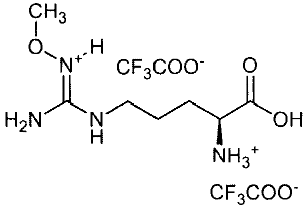

Das geschützte L-Arginin **3** (0,4 mmol) wird in 8 mL TFA und 2,4 mL Thioanisol für 30 min bei Raumtemperatur gerührt. Anschließend wird der Großteil an TFA i. Vak. abdestilliert und 5 mL Wasser sowie 15 mL Diethylether werden zugegeben. Die organische Phase wird noch zweimal mit 5 mL Wasser extrahiert und die vereinigten wässrigen Phasen abschließend mit 5 mL Diethylether gewaschen. Die wässrige Phase wird am Rotationsverdampfer einkonzentriert (bei ca. 35 °C) und mit wenig 0,1 %-iger TFA (in *Aqua bidest.*) aufgenommen. Es folgt die Flashchromatographie über eine RP-18-Säule (Elutionsmittel: 0,1 % TFA_{(aq)}), wobei Ninhydrin-positive Fraktionen vereinigt werden. Die vereinigten Fraktionen werden bis zu einem Volumen von ca. 10 mL am Rotationsverdampfer eingeengt und dann in der Gefriertrocknung lyophilisiert.

Ausbeute: 171 mg eines farblosen Öls (99 %), R_{f} = 0,56 (*i*-Propanol/Wasser/Eisessig, 6:3:1; Ninhydrin)
¹H-NMR (D₂O):
δ/ppm = 1.78 (m, 2H, γ-CH₂), 1.99 (m, 2H, β-CH₂), 3.31 (t, 2H, ³*J* = 6.8 Hz, N-CH₂), 3.75 (s, 3H, O-CH₃), 4.06 (t, ³*J* = 6.2 Hz, 1H, α-CH).
¹³C-NMR (D₂O, TPS):
δ/ppm = 26.4 (γ-CH₂), 29.7 (β-CH₂), 43.0 (N-CH₂), 55.5 (α-CH), 67.3 (O-CH₃), 160.1 (C=N), 174.8 (CO).
HRMS (m/z):
berechnet für C₇H₁₇N₄O₃ [M + H]⁺ = 205.12952, gefunden: 205.12951.

### N^{ω}-Methoxy-L-arginin-ethylester Dihydrochlorid (4)

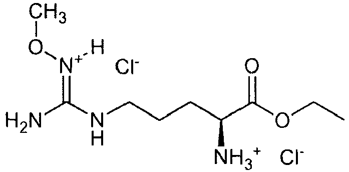

Zur Veresterung werden 238 mg der freien Aminosäure 3 (0,55 mmol) in 5 mL absolutem Ethanol unter Argon-Atmosphäre gelöst. Die Lösung wird 30 min bei -10 °C gerührt, bevor für ca. 5-10 min HCl-Gas in die Lösung eingeleitet wird. Anschließend wird der Ansatz für eine Stunde bei 0 °C weitergerührt und 36 Stunden in den Kühlschrank gestellt. Die Lösung wird vorsichtig i. Vak. bei Raumtemperatur einkonzentriert und lyophilisiert. Das so erhaltene Produkt fällt als sehr hygroskopischer, amorpher Feststoff an, welcher bei Luftkontakt verflüssigt.

| | |
|---|---|
| Ausbeute: | 168 mg eines klaren Öls (99 %) |
| DC: | R_{f} = 0,18 (*i*-Propanol/Wasser/Eisessig, 8:1:1;Ninhydrin) |

¹H-NMR (DMSO-*d*₆):
δ/ppm = 1.24 (t, ³*J* = 7.2 Hz, 3H, CH₂-CH₃), 1.47-1.90 (m, 4H, β,γ-CH₂), 3.22 (m, 2H, N-CH₂), 3.64 (s, 3H, O-CH₃), 3.99 (m, 1H, α-CH), 4.21 (q, ³*J* = 7.2 Hz, 2H, CH₂-CH₃), 8.04 (br s, 2H, NH₂), 8.31 (br t, 1H, NH), 8.72 (br s, 3H, NH₃⁺), 11.33 (br s, 1H, NH⁺). ¹³C-NMR (DMSO-*d*₆):
δ/ppm = 13.9 (CH₂-CH₃), 24.0 (γ-CH₂), 27.0 (β-CH₂), 40.0 (N-CH₂), 51.4 (α-CH), 61.7 (CH₂-CH₃), 64.4 (O-CH₃), 157.2 (C=N), 169.2 (CO).
HRMS (m/z):
berechnet für C₉H₂₁N₄O₃ [M + H]⁺ = 233.16082, gefunden: 233.16064.
C₉H₂₀N₄O₃·2,0 HCl·0,7 H₂O (317.82)
Ber. C 34.01 H 7.42 N 17.63
Gef. C 33.65 H 7.66 N 18.20

### N^{α}-(t-Butyloxycarbonyl)-N^{ω}-ethoxycarbonyl-N^{ω'}-methoxy-L-arginin-t-butylester (5)

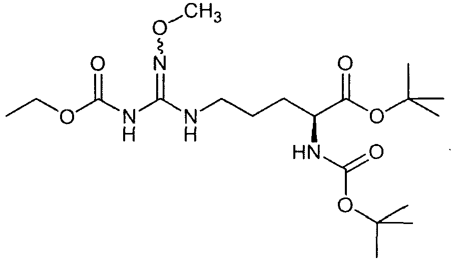

210 mg des Thioharnstoffs 2 (0,5 mmol) werden in 5 mL trockenem Dichlormethan gelöst und 261 µL DIPEA (1,5 mmol) sowie 62,6 mg Methoxylamin Hydrochlorid (0,75 mmol) zugegeben. Die Lösung wird für ca. 30 min auf 0 °C gebracht und 143,5 mg EDCI (0,75 mmol) zugegeben. Das Gemisch wird über Nacht bei Raumtemperatur gerührt. Die Lösung wird mit ca. 10 mL Dichlormethan verdünnt und jeweils mit 5 mL 1 %-iger HCl, Wasser und gesättigter NaCl-Lösung gewaschen. Die organische Phase wird über Na₂SO₄ getrocknet und i. Vak. eingeengt. Das Rohprodukt wird mittels Säulenchromatographie über Kieselgel weiter aufgereinigt (Dichlormethan/Methanol, 98:2).

| | |
|---|---|
| Ausbeute: | 203 mg eines farblosen Öls (94 %) |
| DC: | R_{f} = 0,26 (Dichlormethan/Methanol, 98:2;Ninhydrin) |

¹H-NMR (CDCl₃):
δ/ppm = 1.27 (t, ³*J* = 7.1 Hz, 3H, CH₂-CH₃), 1.43, 1.45 (2 × s, 9H, C(CH₃)₃), 1.56-1.89 (m, 4H, β,γ-CH₂), 3.09 (m, 2H, N-CH₂), 3.66 (s, 3H, O-CH₃), 4.16 (br q, ³*J* = 7.1 Hz, 3H, CH₂-CH₃, α-CH), 5.10, 6.26 (2 × br m, 1H, NH), 7.80 (br s, 1H, NH).
¹³C-NMR (CDCl₃):
δ/ppm = 14.9 (CH₂-CH₃), 25.6 (γ-CH₂), 29.7 (β-CH₂), 29.0, 30.9 (2 × C(CH₃)₃), 41.2 (N-CH₂), 54.5 (α-CH), 62.0 (CH₂-CH₃), 62.6 (O-CH₃), 80.2, 82.5, (2 × C(CH₃)₃), 149.0 (C=N), 153.8 (CO-Eoc), 156.0 (CO-Boc), 172.4 (COO*^{t}*Bu).
MS (ESI):
m/z = 455 [M + Na]⁺, 433 [M + H]⁺, 377 [M - C₄H₈ + H]⁺.
C₁₉H₃₆N₄O₇ (432.52)
Ber. C 52.76 H 8.39 N 12.95
Gef. C 53.65 H 8.57 N 13.24

### N^{ω}-Ethoxycarbonyl-N^{ω'}-methoxy-L-arginin Bis(trifluoracetat) (6)

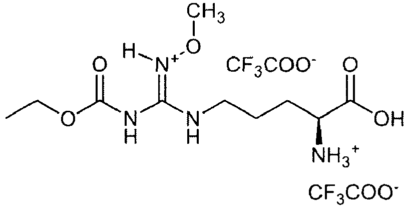

200 mg des vollständig geschützten *N*^{ω}-Methoxy-L-arginins 5 (0,46 mmol) werden in 5 mL TFA zuerst 30 min bei 0 °C und anschließend drei Stunden bei Raumtemperatur gerührt. TFA wird vorsichtig bei möglichst niedriger Temperatur i. Vak. abdestilliert und der Rückstand mit wenig *Aqua bidest.* aufgenommen. Es folgt die weitere Aufreinigung mittels Flashchromatographie auf einer RP-18-Säule (0,1 % TFA in *Aqua bidest.*). Die Ninhydrin-positiven Fraktionen werden vereinigt, am Rotationsverdampfer bei etwa 30 °C auf ein Restvolumen von ca. 10 mL eingeengt und lyophilisiert.

| | |
|---|---|
| Ausbeute: | 225 mg eines farblosen Öls (97 %) |
| DC: | R_{f} = 0,44 (*i*-Propanol/Wasser/Eisessig, 8:1:1,Ninhydrin) |

¹H-NMR (DMSO-*d*₆):
δ/ppm = 1.22 (t, ³*J* = 7.1 Hz, 3H, CH₂-CH₃), 1.50-1.87 (m, 4H, β,γ-CH₂), 3.12 (br t, 2H, N-CH₂), 3.62 (s, 3H, O-CH₃), 3.90 (m, 1H, α-CH), 4.13 (q, ³*J* = 7.1 Hz, 2H, CH₂-CH₃), 7.35 (br s, 1H, NH), 8.28 (br s, 3H, NH₃⁺).
¹³C-NMR (DMSO-*d*₆):
δ/ppm = 14.1 (CH₂-CH₃), 24.6 (γ-CH₂), 27.3 (β-CH₂), 40.6 (N-CH₂), 51.7 (α-CH), 61.6 (CH₂-CH₃), 62.1 (O-CH₃), 149.0 (C=N), 153.5 (CO-Eoc), 170.9 (CO).
HRMS (m/z):
berechnet für C₁₀H₂₁N₄O₅ [M + H]⁺ = 277.15065, gefunden: 277.15049.
C₁₀H₂₀N₄O₅·2,0 CF₃COOH·0.4 H₂O (513.56)
Ber. C 32.74 H 4.87 N 10.91
Gef. C 32.44 H 4.69 N 10.53

### N^{ω}-Ethoxycarbonyl-N^{ω'}-methoxy-L-arginin-ethylester Dihydrochlorid (7)

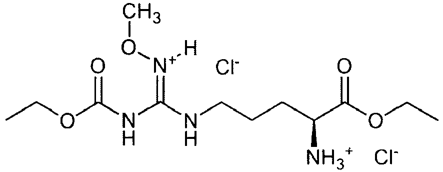

Zur Veresterung werden 200 mg der freien Aminosäure 6 (0,397 mmol) in 5 mL absolutem Ethanol unter Argon-Atmosphäre gelöst. Die Lösung wird 30 min bei -10 °C gerührt, bevor für ca. 5-10 min HCl-Gas in die Lösung eingeleitet wird. Anschließend wird der Ansatz für eine Stunde bei 0 °C weitergerührt und 36 Stunden in den Kühlschrank gestellt. Die Lösung wird vorsichtig i. Vak. bei Raumtemperatur einkonzentriert und lyophilisiert. Das so erhaltene Produkt fällt als sehr hygroskopischer, amorpher Feststoff an, welcher bei Luftkontakt verflüssigt.

Ausbeute: 150 mg eines farblosen Öls (99 %)
¹H-NMR (DMSO-*d*₆):
δ/ppm = 1.23, 1.25 (2 × t, *³J* = 7.0 Hz, 3H, CH₂-CH₃), 1.55-1.88 (m, 4H, β,γ-CH₂), 3.32 (br t, 2H, N-CH₂), 3.71 (s, 3H, O-CH₃), 3.95 (m, 1H, α-CH), 4.19 (m, 4H, 2 × CH₂-CH₃), 8.80 (br s, 4H, NH₃⁺, NH).
¹³C-NMR (DMSO-*d*₆):
δ/ppm = 13.9, 14.0 (2 × CH₂-CH₃), 24.0 (γ-CH₂), 26.9 (β-CH₂), 41.1 (N-CH₂), 51.4 (α-CH₂), 61.7, 62.5 (2 × CH₂-CH₃), 63.9 (O-CH₃), 150.7 (C=N), 152.6 (CO-Eoc), 169.2 (COOEt).
HRMS (m/z):
berechnet für C₁₂H₂₅N₄O₅ [M + H]⁺ = 305.18195, gefunden: 305.18176.
C₁₂H₂₄N₄O₅·2,0 HCl·0,6 H₂O (388.08)
Ber. C 37.14 H 7.06 N 14.44
Gef. C 36.67 H 7.59 N 15.00

### Ausführungsbeispiel 2: O-Carboxyalkylierte NOHA-Derivate

### N^{ω}-Benzyloxycarbonyl-N^{α}-(t-butyloxycarbonyl)-N^{ω'}-(methoxycarbonyl)methoxy-L-arginin-t-butylester (8)

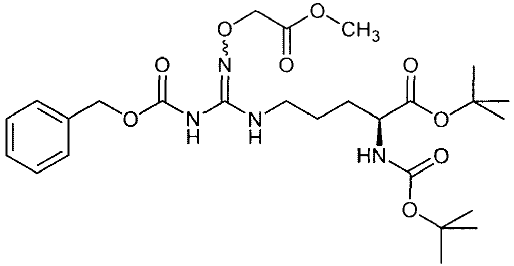

241 mg des Thioharnstoffs 1 (0,5 mmol) werden in 5 mL trockenem Dichlormethan gelöst und 261 µL DIPEA (0,75 mmol) sowie 79 mg Aminooxyessigsäuremethylester (0,75 mmol) zugegeben. Die Lösung wird für ca. 30 min auf 0 °C gebracht und 143,5 mg EDCI (0,75 mmol) werden zugegeben. Das Gemisch wird über Nacht bei Raumtemperatur gerührt. Die Lösung wird mit ca. 10 mL Dichlormethan verdünnt und jeweils mit 5 mL 1 %-iger HCl, Wasser und gesättigter NaCl-Lösung gewaschen. Die organische Phase wird über Na₂SO₄ getrocknet und i. Vak. eingeengt. Das Rohprodukt wird mittels Chromatographie über Kieselgel weiter aufgereinigt (Cyclohexan/Ethylacetat, 3:2).

| | |
|---|---|
| Ausbeute: | 254 mg eines farblosen Öls (92 %) |
| DC: | R_{f} = 0,48 (Cyclohexan/Ethylacetat, 3:2;Ninhydrin) |

¹H-NMR (CDCl₃):
δ/ppm = 1.44, 1.46 (2 × s, 9H, C(CH₃)₃), 1.50-1.87 (m, 4H, β,γ-CH₂), 3.07 (m, 2H, N-CH₂), 3.73 (s, 3H, O-CH₃), 4.16 (m, 1H, α-CH), 4.41 (s, 2H, O-CH₂), 5.08 (m, 1H, NH), 5.15 (s, 2H, CH₂-Cbz), 6.37 (br t, ³*J* = 5.3 Hz, 1H, NH), 7.30-7.39 (m, 5H, ArH), 8.23 (br s, 1H, NH).
¹³C-NMR (CDCl₃):
δ/ppm = 25.5 (γ-CH₂), 28.7 (β-CH₂), 29.0, 30.9 (2 × C(CH₃)₃), 41.2 (N-CH₂), 52.4 (O-CH₃), 54.5 (α-CH), 68.3 (CH₂-Cbz), 71.2 (O-CH₂), 80.3, 82.5 (2 × C(CH₃)₃), 129.0, 129.28, 129.34
(ArCH), 135.9 (ArC), 150.8 (C=N), 153.7 (CO-Cbz), 156.0 (CO-Boc), 171.7, 172.4 (COO*^{t}*Bu, COOMe).
MS (ESI):
m/z = 575 [M + Na]⁺, 553 [M + H]⁺, 497 [M - C₄H₈ + H]⁺.

### N^{ω}-Carboxymethoxy-L-arginin Dihydrochlorid (9)

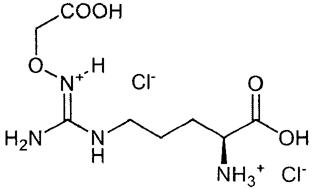

Es werden 270 mg des vollständig geschützten *N*^{ω}-Carboxymethoxy-L-arginins 8 (0,489 mmol) in 5 mL 6 N HCl für vier Stunden bei 50-60 °C gerührt. Der Ansatz wird i. Vak. eingeengt, mit ca. 1-2 mL *Aqua bidest.* versetzt und anschließend mittels Flashchromatographie auf einer RP-18-Säule gereinigt (0,1 % TFA in *Aqua bidest.*)*.* Ninhydrin-positive Fraktionen werden vereinigt, bis auf wenige Milliliter am Rotationsverdampfer bei 30 °C eingeengt und im Anschluss lyophilisiert.

| | |
|---|---|
| Ausbeute: | 150 mg eines weißen, amorphen Feststoffes (96 %) |
| DC: | R_{f} = 0,53 (*i*-Propanol/Wasser/Eisessig, 6:3:1; Ninhydrin) |

¹H-NMR (D₂O):
δ/ppm = 1.76-2.18 (m, 4H, β,γ-CH₂), 3.41 (br t, ³*J* = 6.7 Hz, 2H, N-CH₂), 4.17 (br t, ³*J* = 6.2 Hz, α-CH), 4.63 (s, 2H, O-CH₂).
¹³C-NMR (D₂O, TPS):
δ/ppm = 26.3 (γ-CH₂), 29.6 (β-CH₂), 43.2 (N-CH₂), 55.3 (α-CH), 75.5 (O-CH₂), 161.0 (C=N), 174.6, 175.3 (2 × CO).
MS (ESI):
m/z = 249 [M + H]⁺.
C₈H₁₆N₄O₅·2,0 HCl·0,5 H₂O (330.17)
Ber. C 29.10 H 5.80 N 16.97
Gef. C 29.00 H 5.99 N 17.16

### N^{α}-(t-Butyloxycarbonyl)-N^{ω}-ethoxycarbonyl-N^{ω'}-(ethoxycarbonyl)methoxy-L-arginin-t-butylester (10)

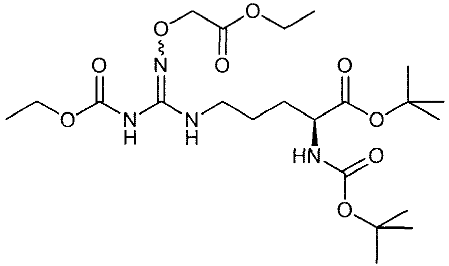

Die Darstellung und Aufarbeitung erfolgt unter denselben Bedingungen wie sie für die analoge Verbindung 8 beschrieben sind, mit dem Thioharnstoff 2 und Aminooxyessigsäureethylester als Ausgangssubstanzen (Ansatzgröße: 1,0 mmol).

| | |
|---|---|
| Ausbeute: | 500 mg eines farblosen Öls (99 %) |
| DC: | R_{f} = 0,51 (Cyclohexan/Ethylacetat, 3:2; Ninhydrin) |

¹H-NMR (CDCl₃):
δ/ppm = 1.26 (t, ³*J* = 7.1 Hz, 3H, CH₂-CH₃), 1.27 (t, ³*J* = 7.1 Hz, 3H, CH₂-CH₃), 1.42, 1.44 (2 × s, 9H, C(CH₃)₃), 1.51-1.85 (m, 4H, β,γ-CH₂), 3.06 (m, 2H, N-CH₂), 4.16 (q, ³*J* = 7.11 Hz, 2H, CH₂-CH₃), 4.19 (q, ³*J* = 7.17 Hz, 2H, CH₂-CH₃), 4.39 (s, 2H, O-CH₂), 5.08 (m, 1H, NH), 6.40 (br t, 1 H, NH), 8.19 (br s, 1 H, NH).
¹³C-NMR (CDCl₃):
δ/ppm = 14.1, 14.2 (2 × CH₂-CH₃), 24.8 (γ-CH₂), 27.9, 28.3 (2 × C(CH₃)₃), 30.2 (β-CH₂), 40.5 (N-CH₂), 53.7 (α-CH), 60.8, 61.9 (2 × CH₂-CH₃), 70.7 (O-CH₂), 79.5, 81.8 (2 × C(CH₃)₃), 150.4 (C=N), 153.2 (CO-Eoc), 155.3 (CO-Boc), 170.7, 171.7 (COOEt, COO*^{t}*Bu).
MS (ESI):
m/z = 527 [M + Na]⁺, 505 [M + H]⁺, 449 [M - C₄H₈]⁺.
C₂₂H₄₀N₄O₉ (504.59)
Ber. C 52.37 H 7.99 N 11.10
Gef. C 53.09 H 7.90 N 11.44

### N^{ω}-Ethoxycarbonyl-N^{ω'}-(ethoxycarbonyl)methoxy-L-arginin Bis(trifluoracetat) (11)

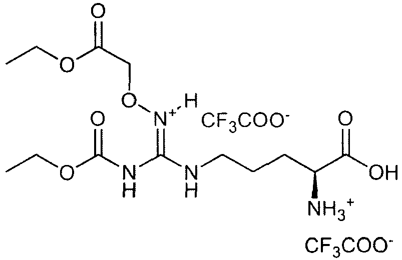

Es werden 252 mg der vollständig geschützten Vorstufe 10 (0,5 mmol) in 5 mL TFA zuerst 30 min bei 0 °C und anschließend drei Stunden bei Raumtemperatur gerührt. TFA wird vorsichtig bei möglichst niedriger Temperatur i. Vak. abdestilliert und der Rückstand mit wenig *Aqua bidest.* aufgenommen. Es folgt die weitere Aufreinigung mittels Flashchromatographie auf einer RP-18-Säule (0,1 % TFA_{(aq)}/Methanol-Stufengradient, 5-30 %). Die Ninhydrin-positiven Fraktionen werden vereinigt, am Rotationsverdampfer bei ca. 30 °C auf ein Restvolumen von ca. 10 mL eingeengt und lyophilisiert.

| | |
|---|---|
| Ausbeute: | 277 mg eines klaren Öls (96 %) |
| DC: | R_{f} = 0,62 (*i*-Propanol/Wasser/Eisessig, 8:1:1; Ninhydrin) |

In DMSO-*d*₆ als Lösungsmittel liegt die Substanz als Isomerengemisch vor, im Verhältnis von ca. 8.6:1.4 (bei 300 K, bezogen auf das Singulett von CH₂ des Ethoxycarbonylmethoxy-Rests). Die angegebenen Verschiebungen beziehen sich auf das Hauptisomer.
¹H-NMR (DMSO-*d*₆):
δ/ppm = 1.19 (t, ³*J* = 7.10 Hz, 3H, CH₂-CH₃), 1.21 (t, ³*J* = 7.08 Hz, 3H, CH₂-CH3), 1.48-1.87 (m, 4H, β,γ-CH₂), 3.02 (m, 2H, N-CH₂), 3.89 (m, 1H, α-CH), 4.10 (q, ³*J* = 7.04 Hz, 2H, CH₂-CH₃), 4.12 (q, ³*J* = 7.13 Hz, 2H, CH₂-CH₃), 4.37 (s, 2H, O-CH₂), 6.54 (br s, 1H, NH), 8.24 (br s, 3H, NH₃⁺), 10.64 (br s, COOH).
¹³C-NMR (DMSO-*d*₆):
δ/ppm = 14.0, 14.2 (2 × CH₂-CH₃), 27.4 (CH₂), 40.3 (N-CH₂), 51.8 (α-CH), 60.2, 61.3 (2 × CH₂-CH₃), 70.3 (O-CH₂), 169.9, 171.0 (3 × CO).
HRMS (m/z):
berechnet für C₁₃H₂₅N₄O₇ [M + H]⁺ = 349.17178, gefunden: 349.17155.
C₁₃H₂₄N₄O₇·3,2 CF₃COOH·1,5 H₂O (740.26)
Ber. C 31.48 H 4.11 N 7.57
Gef. C 31.52 H 4.24 N 7.40

### N^{ω}-Ethoxycarbonyl-N^{ω'}-(ethoxycarbonyl)methoxy-L-arginin-ethylester Dihydrochlorid (12)

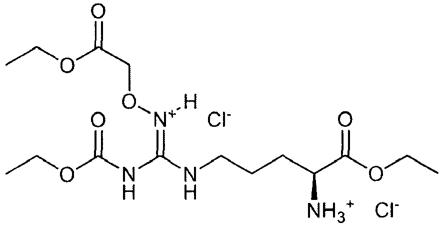

Zur Veresterung werden 288 mg der freien Aminosäure 11 (0,5 mmol) in 5 mL absolutem Ethanol unter Argon-Atmosphäre gelöst. Die Lösung wird 30 min bei -10 °C gerührt, bevor für ca. 5-10 min HCl-Gas in die Lösung geleitet wird. Anschließend wird der Ansatz für eine Stunde bei 0 °C weitergerührt und über Nacht in den Kühlschrank gestellt. Die Lösung wird vorsichtig i. Vak. bei Raumtemperatur einkonzentriert, lyophilisiert und liefert einen stark hygroskopischen, amorphen Feststoff, der bei Luftkontakt verflüssigt.

| | |
|---|---|
| Ausbeute: | 222 mg eines farblosen Öls (99 %) |
| DC: | R_{f} = 0,57 (*i*-Propanol/Wasser/Eisessig, 8:1:1; Ninhydrin) |

¹H-NMR (DMSO-*d*₆):
δ/ppm = 1.20 (t, ³*J* = 7.16 Hz, 3H, CH₂-CH₃), 1.23 (t, ³*J* = 7.13 Hz, 3H, CH₂-CH₃), 1.26 (t, ³*J* = 7.10 Hz, 3H, CH₂-CH₃), 1.51-1.87 (m, 4H, β,γ-CH₂), 3.17 (m, 2H, N-CH₂), 3.95 (m, 1H, α-CH), 4.14 (q, ³*J* = 7.12 Hz, 2H, CH₂-CH₃), 4.15 (q, ³*J* = 7.10 Hz, 2H, CH₂-CH₃), 4.17-4.25 (m, 2H, CH₂-CH₃), 4.51 (s, 2H, O-CH₂), 7.80 (br s, 1H, NH), 8.55 (br s, 1H, NH), 8.72 (br s, 3H, NH₃⁺).
¹³C-NMR (DMSO-*d*₆):
δ/ppm = 13.9, 14.0, 14.1 (3 × CH₂-CH₃), 24.2 (γ-CH₂), 27.2 (β-CH₂), 40.8 (N-CH₂), 51.5 (α-CH), 60.5, 61.7, 61.9 (3 × CH₂-CH₃), 71.1 (O-CH₂), 150.3 (C=N), 153.0 (CO-Eoc), 169.1, 169.3 (2 × CO).
HRMS (m/z):
berechnet für C₁₅H₂₉N₄O₇ [M + H]⁺ = 377.20308, gefunden: 377.20287.

### Ausführungsbeispiel 3: O-glycosidisch konjugierte NOHA-Derivate

### N^{α}-(t-Butyloxycarbonyl)-N^{ω}-ethoxycarbonyl-N^{ω'}-(2,3,4,6-tetra-O-acetyl-β-D-galactopyranos-1-yl)oxy-L-arginin-t-butylester (13)

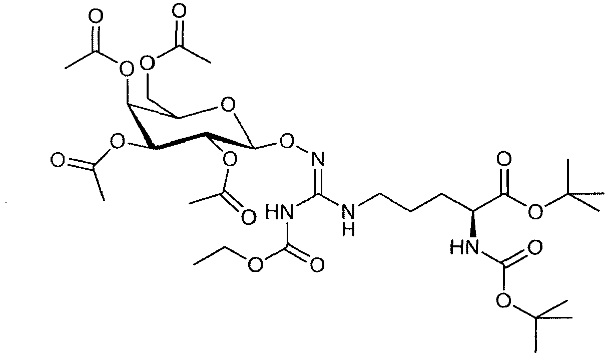

Es werden 210 mg des Thioharnstoffs 2 (0,5 mmol), 218 mg 1-Aminooxy-2,3,4,6-tetra-*O-*acetyl-β-D-galactopyranose (0,6 mmol) und 104,5 ul DIPEA (0,6 mmol) in 5 mL trockenem Dichlormethan gelöst. Der Ansatz wird auf 0 °C gekühlt, 115 mg EDCI (0,6 mmol) werden zugegeben und 48 Stunden bei Raumtemperatur gerührt. Die Lösung wird mit ca. 10 mL Dichlormethan verdünnt und jeweils mit 5 mL 1 %-iger HCl, Wasser und gesättigter NaCl-Lösung gewaschen. Die organische Phase wird über Na₂SO₄ getrocknet und i. Vak. eingeengt. Das Rohprodukt wird mittels Säulenchromatographie über Kieselgel weiter aufgereinigt (Dichlormethan/Methanol, 97:3). Die vereinigten Fraktionen des gereinigten Produkts werden i. Vak. zu einem klaren Öl einkonzentriert. Wiederholte Zugabe und Entfernung von trockenem Dichlormethan liefert einen festen weißen Schaum, der bei Luftkontakt verflüssigt.

| | |
|---|---|
| Ausbeute: | 262 mg eines farblosen Öls (70 %) |
| DC: | R_{f} = 0,35 (Dichlormethan/Methanol, 97:3; Ninhydrin) |

¹H-NMR (CDCl₃):
δ/ppm = 1.29 (t, ³*J* = 7.1 Hz, 3H, CH₂-CH₃), 1.43, 1.45 (2 × s, 9H, C(CH₃)₃), 1.52-1.86 (m, 4H, β,γ-CH₂), 1.98, 2.02, 2.05, 2.13 (4 × COCH₃), 3.08 (m, 2H, N-CH₂), 3.96 (br t, ³*J* = 6.8 Hz, 1H, 5'-CH), 4.11-4.21 (m, 5H, α-CH, CH₂-CH₃, 3'-CH, NH), 4.85 (d, ³*J* = 8.3 Hz, 1H, 1'-CH), 5.06 (m, 2H, 6'-CH₂), 5.25 (dd, ³*J* = 10.4, 8.3 Hz, 1H, 2'-CH), 5.39 (dd, ³*J* = 3.5, 1.0 Hz, 1H, 4'-CH), 6.47 (br t, 1H, NH), 7.65 (br s, 1H, NH).
MS (ESI):
m/z = 772 [M + Na]⁺, 750 [M + H]⁺, 707 [M - C₂H₂O + H]⁺.

### Natriumsalz des N^{ω}-Ethoxycarbonyl-N^{ω'}-(2,3,4,6-tetra-O-acetyl-β-D-galactopyranos-1-yl)oxyL-arginins (14)

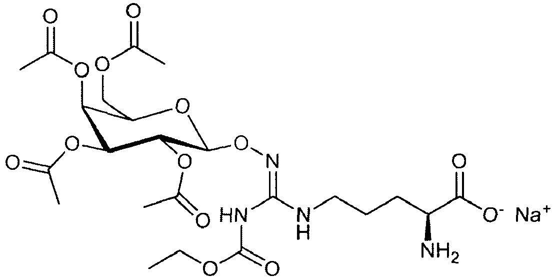

In einem Schlenk-Kolben werden 120 mg (0,16 mmol) der vollständig geschützten und sorgfältig getrockneten Vorstufe **13** unter Argon-Begasung in ca. 10 mL trockenem Diethylether gelöst. Die Lösung wird für ca. 30 min bei -15 °C gerührt und anschließend wird unter gleichzeitiger Argon-Begasung für ca. 5 min HCl-Gas vorsichtig in die Lösung geleitet. Das Reaktionsgemisch wird über Nacht in den Kühlschrank gestellt und am folgenden Tag zur Aufarbeitung i. Vak. eingeengt. Der weiß-gelbe Feststoff wird dann mit ca. 1-2 mL 0,5 M NaHCO₃-Lösung aufgenommen und mittels Flashchromatographie über eine RP-18-Säule aufgereinigt (Fließmittel: *Aqua bidest.*/*Methanol*), wobei ein Stufengradient für die Elution verwendet wird. Es wird mit 10 % Methanol-Anteil begonnen, dann auf 25 % und abschließend auf 50 % Methanol erhöht. Die produkthaltigen Fraktionen werden vereinigt, bei 30 °C auf ein Restvolumen von ca. 50 mL i. Vak. einkonzentriert und anschließend lyophilisiert.

Ausbeute: 74 mg eines feinen weißen Pulvers (75 %)

In D₂O als Lösungsmittel liegt die Substanz als Isomerengemisch vor, im Verhältnis von ca. 6:4 (bei 300 K, bezogen auf das Triplett von CH₃ der Ethoxycarbonyl-Funktion). Die angegebenen Verschiebungen beziehen sich auf das Hauptisomer.

¹H-NMR (D₂O, TPS): δ/ppm = 1.28 (t, ³*J* = 7.1 Hz, 3H, CH₂-CH₃), 1.52-1.97 (m, 4H, β,γ-CH₂), 2.02, 2.08, 2.12, 2.23 (4 × s, 3H, COCH₃), 3.16 (br t, ³*J* = 6.8 Hz, 2H, 6'-CH₂), 3.75 (t, ³*J* = 6.3 Hz, 1H, 5'-CH), 4.15-4.30 (m, 5H, CH₂-CH₃, α-CH, N-CH₂), 5.07 (d, ³*J* = 8.0 Hz, 1H, 1'-CH), 5.21-5.33 (m, 2H, 2',3'-CH), 5.47 (m, 1H, 4'-CH).
¹³C-NMR (D₂O, TPS):
δ/ppm = 16.2 (CH₂-CH₃), 22.7, 22.8, 22.9, (4 × COCH₃), 26.6 (γ-CH₂), 30.6 (β-CH₂), 42.7 (N-CH₂), 57.2 (α-CH), 64.6 (6'-CH₂), 65.8 (CH₂-CH₃), 70.6, 70.7, 73.4, 74.1 (2',3',4',5'-CH), 104.1 (1'-CH), 155.6 (C=N), 156.8 (CO-Eoc), 175.4, 175.8, 176.2, 177.1 (4 × COCH₃). MS (ESI):
m/z = 615 [M + Na]⁺, 593 [M + H]⁺, 551 [M - C₂H₂O + H]⁺, 331 [C₁₄H₁₉O₉]⁺.
HRMS (m/z):
berechnet für C₂₃H₃₃N₄O₁₄Na [M + Na]⁺ = 615.21202, gefunden: 615.21164
C₂₃H₃₅N₄NaO₁₄ (614.53)
Ber. C 44.95 H 5.74 N 9.12
Gef. C 45.08 H 6.21 N 8.77

## Patentansprüche

1. Verwendung eines Stoffs mit der allgemeinen Formel (I) 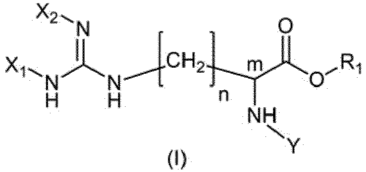 zur Herstellung eines Arzneimittels zur Behandlung einer mit einer Stickstoffmonoxid-Defizienz einhergehenden Krankheit,
wobei
X₁, X₂ ausgewählt sind aus der Gruppe von Substituenten bestehend aus 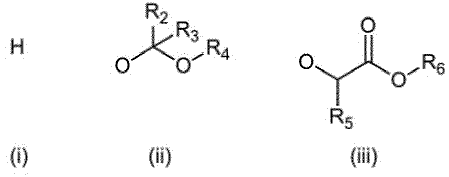 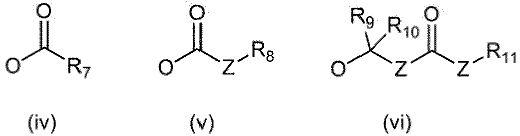 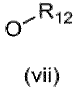 wobei X₁ und X₂ verschieden oder mit Ausnahme von Wasserstoff (i) identisch sind;
R₁ Wasserstoff, ein Alkyl- oder ein Arylrest ist;
R₂, R₃ und R₄ Wasserstoff, Alkyl- oder Arylrest sind oder R₂, R₃ und/oder R₄ ein Monosaccharid oder ein Monosaccharidderivat ist,
R₅-R₁₂ ein Wasserstoff, Alkyl- oder Arylrest ist;
Y Wasserstoff, ein Alkoxycarbonyl- oder ein Aryloxycarbonylrest ist;
Z Sauerstoff (O) oder Stickstoff (N) ist;
n 2 oder 3 ist;
und wobei X₁ nicht (i) ist, wenn X₂ =(vii) und R₁₂=H sind und wobei *N*^{ω}-Hydroxy-L-arginin und dessen Carbonsäureester ausgenommen sind oder
X₁ und X₂ ein Ringsystem der Struktur (viii) oder (ix) ausbilden: 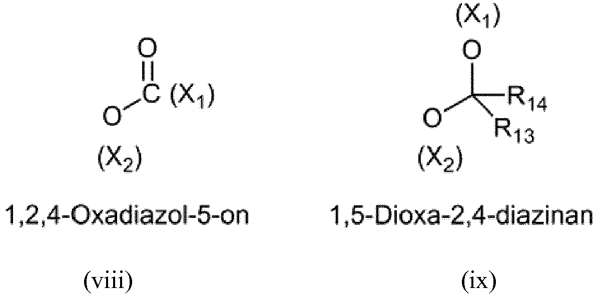 wobei R₁₃, R₁₄ Wasserstoff, Alkyl- oder Arylreste sind.

2. Verwendung nach Anspruch 1, **dadurch gekennzeichnet, dass** R₂, R₃ und/oder R₄ ein Monosaccharid oder ein Monosaccharidderivat ist.

3. Verwendung nach Anspruch 2, wobei der Stoff N^{α}-(t-Butyloxycarbonyl)-N^{ω}-ethoxycarbonyl-N^{ω'}-(2,3,4,6-tetra-O-acetyl-β-D-galactopyranos-1-yl)oxy-L-arginin-t-butylester oder Natriumsalz des Nω-Ethoxycarbonyl-N^{ω'}(2,3,4,6-tetra-O-acetyl-β-D-galactopyranos-1-yl)oxy-L-arginins ist.

4. Verwendung nach einem der vorhergehenden Ansprüche zur Prophylaxe und/oder Behandlung von kardiovaskulären Erkrankungen wie Bluthochdruck, Herzinsuffizienz, stabiler und instabiler Angina pectoris, peripheren und kardialen Gefäßerkrankungen, Arrhythmien, thromboembolischer Erkrankungen und Ischämien wie Myokardinfarkt, Hirnschlag, transistorischen und ischämischen Attacken, peripheren Durchblutungsstörungen oder zur Verhinderung von Restenosen nach Thrombolysetherapien, percutan transluminalen Angioplastien, percutan transluminalen Koronarangioplastien, Bypass, oder zur Behandlung von Atherosklerose.

5. Verwendung nach einem der Ansprüche 1 bis 4 zur Prophylaxe und/oder Behandlung von Krankheiten des Urogenitalsystems, erektiler Dysfunktion, weiblicher sexueller Dysfunktion oder Inkontinenz.

6. Verwendung nach einem der Ansprüche 1 bis 3 zur Prophylaxe und/oder Behandlung von malignen Tumoren.

7. Verwendung nach einem der Ansprüche 1 bis 3 zur Prophylaxe und/oder Behandlung von das NO/cGMP-System im Zentralnervensystem störenden Krankheiten.

8. Verwendung nach Anspruch 7 zur Verbesserung kognitiver Defizite, Lern- und Gedächtnisleistungen, zur Behandlung der Alzheimerschen Krankheit, zur Behandlung von Angst-, Spannungs- und Depressionszuständen, zentralnervös bedingten Sexualdysfunktionen und Schlafstörungen, zur Regulierung krankhafter Störungen der Nahrungs-, Genuss- und Suchtmittelaufnahme, oder zur Regulation der cerebralen Durchblutung als Mittel zur Bekämpfung der Migräne oder anderer Formen des Kopfschmerzes.

## Claims

1. Use of a substance having the general formula (I) 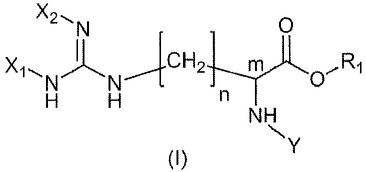 for producing a pharmaceutical substance for treating a disease accompanying a nitrogen-monoxide deficiency,
wherein
X₁, X₂ are selected from the group of substituents consisting of 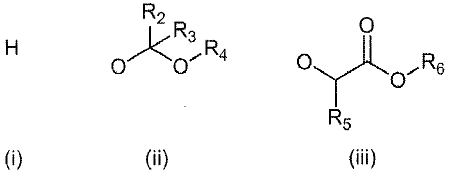 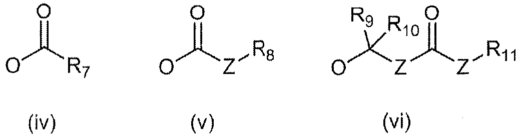 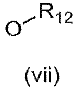 wherein X₁ and X₂ are different or identical with the exception of hydrogen (i);
R₁ is hydrogen, an alkyl or an aryl group;
R₂, R₃ and R₄ are hydrogen, alkyl or aryl group or R₂, R₃ and/or R₄ is a monosaccharide or a monosaccharide derivate,
R₅-R₁₂ is hydrogen, alkyl or aryl group;
Y is hydrogen, an alkoxycarbonyl or an aryloxycarbonyl group;
Z is oxygen (O) or nitrogen (N);
n is 2 or 3;
and wherein X₁ is not (i) if X₂=(vii) and R₁₂=H and wherein *N*^{ω}-hydroxy-L-arginine and its carboxylic acid ester are exempt or
X₁ and X₂ form a ring system having the structure (viii) or (ix): 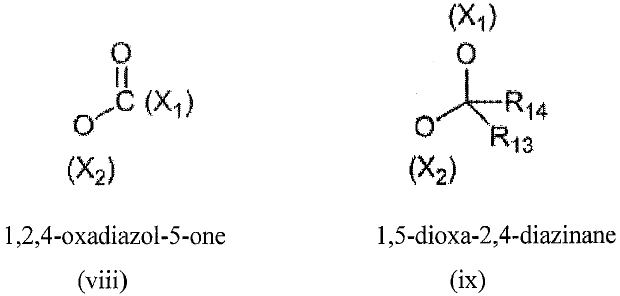 wherein R₁₃, R₁₄ are hydrogen, alkyl or aryl groups.

2. Use according to claim 1, **characterised in that** R₂, R₃ and/or R₄ is a monosaccharide or a monosaccharide derivate.

3. Use according to claim 2, wherein the substance is N^{α}-(t-butyloxycarbonyl)-N^{ω}-ethoxycarbonyl-N^{ω'}-(2,3,4,6-tetra-O-acetyl-β-D-galactopyranos-1-yl)oxy-L-arginine-t-butylester or a sodium salt of Nω-ethoxycarbonyl-N^{ω'}-(2,3,4,6-tetra-O-acetyl-β-D-galactopyranos-1-yl)oxy-L-arginine.

4. Use according to any one of the preceding claims for the prophylaxis and/or treatment of cardiovascular diseases such as high blood pressure, heart failure, stable and unstable angina pectoris, peripheral and cardial vessel diseases, arrhythmias, thromboembolic diseases and ischemias such as myocardial infarcation, stroke, transient and ischemic attacks, peripheral circulatory disorders or for the prevention of restenoses after thrombolytic therapies, percutaneous transluminal angioplasties, percutaneous transluminal coronary angioplasties, bypass or for the treatment of atherosclerosis.

5. Use according to any one of claims 1 to 4 for the prophylaxis and/or the treatment of diseases of the urogenital system, erectile dysfunction, female sexual dysfunction or incontinence.

6. Use according to any one of claims 1 to 3 for the prophylaxis and/or the treatment of malign tumors.

7. Use according to any one of claims 1 to 3 for the prophylaxis and/or the treatment of diseases interfering the NO/cGMP system in the central nervous system.

8. Use according to claim 7 for improving cognitive deficits, learning and memory efficiency, for the treatment of Alzheimer's disease, for the treatment of states of fear, tension and depression, sexual dysfunctions and sleeping disorders caused by the central nervous system, for regulating pathological disorders of the food, stimulant and addictive substance intake or for regulating the cerebral perfusion as means for fighting migraine or other forms of headache.

## Revendications

1. Utilisation d'une substance avec la formule générale (I) 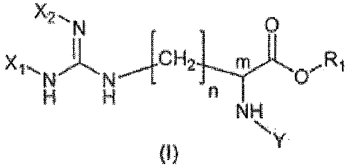 pour la préparation d'un médicament destiné au traitement d'une maladie s'accompagnant d'une insuffisance en monoxyde d'azote,
dans laquelle
X₁, X₂ sont choisis dans le groupe de substituants constitué de 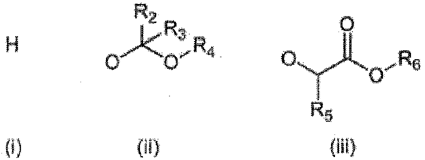 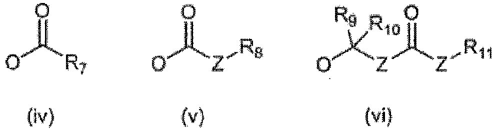 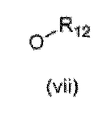
dans laquelle X₁, X₂ sont différents ou identiques à l'exception de l'hydrogène (i) ;
R₁ est l'hydrogène, un résidu alkyle ou aryle ;
R₂, R₃ et R₄ sont l'hydrogène, un résidu alkyle ou aryle ou R₂, R₃ et/ou R₄ sont un monosaccharide ou un dérivé de monosaccharide,
R₅ - R₁₂ sont l'hydrogène, un résidu alkyle ou aryle ;
Y est l'hydrogène, un résidu alcoxycarbonyle ou aryloxycarbonyle ;
Z est l'oxygène (O) ou l'azote (N) ;
n est 2 ou 3 ;
et dans laquelle X₁ n'est pas (i), si X₂ =(vii) et R₁₂=H et dans laquelle la N^{ω}-hydroxy-_{L}-arginine et ses esters d'acide carboxylique sont exclus ou
X₁ et X₂ constituent un système cyclique de structure (viii) ou (ix) : 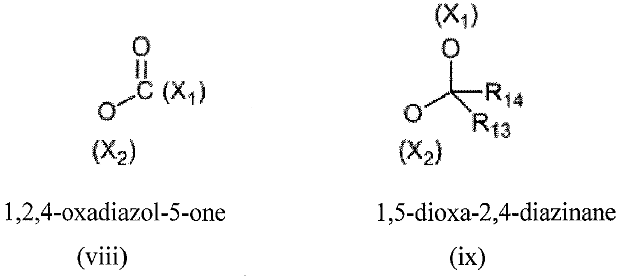 dans lesquelles R₁₃, R₁₄ sont l'hydrogène, des résidus alkyle ou aryle.

2. Utilisation selon la revendication 1, **caractérisée en ce que** R₂, R₃ et/ou R₄ sont un monosaccharide ou un dérivé de monosaccharide.

3. Utilisation selon la revendication 2, dans laquelle la substance est l'ester t-butylique de N^{α}-(t-butyloxycarbonyl)-N^{ω}-éthoxycarbonyl-N^{ω'}-(2,3,4,6-tétra-O-acétyl-β-D-galactopyranos-1-yl)oxy-L-arginine ou un sel sodique de la N^{ω}-éthoxycarbonyl-N^{ω'}-(2,3,4,6-tétra-O-acétyl-β-D-galactopyranos-1-yl)oxy-L-arginine.

4. Utilisation selon l'une des revendications précédentes pour la prophylaxie et/ou le traitement des maladies cardiovasculaires comme l'hypertension, l'insuffisance cardiaque, l'angine de poitrine stable et instable, les maladies des vaisseaux périphériques et cardiaques, les arythmies, les maladies thromboemboliques et les ischémies comme l'infarctus du myocarde, l'accident vasculaire cérébral, les attaques transitoires et ischémiques, les troubles de la circulation périphérique ou pour l'inhibition des resténoses après des thérapies par thrombolyse, des angioplasties transluminales percutanées, des angioplasties coronaires transluminales percutanées, un pontage ou pour le traitement de l'athérosclérose.

5. Utilisation selon l'une des revendications 1 à 4 pour la prophylaxie et/ou le traitement des maladies du système génito-urinaire, des troubles de la fonction érectile, des troubles de l'appareil sexuel féminin ou de l'incontinence.

6. Utilisation selon l'une des revendications 1 à 3 pour la prophylaxie et/ou le traitement des tumeurs malignes.

7. Utilisation selon l'une des revendications précédentes 1 à 3 pour la prophylaxie et/ou le traitement de maladies perturbant la voie NO/GMPc dans le système nerveux central.

8. Utilisation selon la revendication 7 pour l'amélioration des déficits cognitifs, des performances d'apprentissage et de la mémoire, pour le traitement de la maladie d'Alzheimer, pour le traitement des états anxieux, stressés et dépressifs, des troubles sexuels conditionnés par le système nerveux central et des troubles du sommeil, pour la régulation de troubles pathologiques liés à la consommation d'aliments, de stimulants et de substances addictives, ou pour la régulation de la circulation sanguine cérébrale comme moyen pour lutter contre la migraine ou d'autres formes de céphalées.
